# EUROPEAN PATENT SPECIFICATION

(11) **EP 1 794 106 B1**
(45) Date of publication and mention of the grant of the patent: **07.04.2010**
(21) Application number: 05777468.9
(22) Date of filing: 20.07.2005
(51) Int. Cl.: C07C 7/20, C07C 309/42

(54) **SULFONATED PHENOLS WITH NITROPHENOLS AS POLYMERIZATION INHIBITORS**
SULFONIERTE PHENOLE MIT NITROPHENOLEN ALS POLYMERISATIONSINHIBITOREN
PHENOLS SULFONES AVEC NITROPHENOLS COMME INHIBITEURS DE POLYMERISATION

(30) Priority: 28.09.2004 US 614378 P; 29.11.2004 US 631241 P; 29.06.2005 US 172517
(43) Date of publication of application: 13.06.2007
(73) Proprietor: Chemtura Corporation, Middlebury, CT 06749 (US)
(72) Inventor: KOSOVER, Vilen, Cheshire, CT 06410 (US); FABIAN, Jesus, R., Wethersfield, CT 06109 (US); LIPPAI, Istvan, Naugatuck, CT 06770 (US); BENAGE, Brigitte, Wolcott, CT 06716 (US); ABRUSCATO, Gerald, J., Southington, CT 06489 (US)
(74) Representative: Schön, Christoph
(86) International application number: PCT/US2005/026016
(87) International publication number: WO 2006/036274

(56) References cited:
- EP-A- 0 532 240
- WO-A-02/33026
- WO-A-03/070687
- GB-A- 1 354 420
- US-A- 4 086 147
- US-A- 4 468 343
- US-A- 4 664 845
- PATENT ABSTRACTS OF JAPAN vol. 018, no. 615 (C-1277), 24 November 1994 (1994-11-24) & JP 06 234700 A (DAINIPPON INK & CHEM INC), 23 August 1994 (1994-08-23)

## Description

### BACKGROUND OF THE INVENTION

### 1. Field of the Invention

The present invention is directed to the inhibition of the polymerization of ethylenically unsaturated monomers by means of the addition thereto of the combination of a sulfonated phenol and a nitrophenol.

### 2. Description of Related Art

Many ethylenically unsaturated monomers undesirably polymerize at various stages of their manufacture, processing, handling, storage, and use. Polymerization, such as thermal polymerization, during their purification results in the loss of the monomer, i.e., a lower yield, and an increase in the viscosity of any tars that may be produced. The processing and handling of the higher viscosity tars then requires higher temperature and work (energy cost) to remove residual monomer.

Polymerization can also result in equipment fouling, especially in the case of production of acrylic monomers. Such polymerization causes loss in production efficiency owing to the deposition of polymer in or on the equipment being used. These deposits must be removed from time to time, leading to additional loss in production of the monomer.

A wide variety of compounds has been proposed and used for inhibiting uncontrolled and undesired polymerization of ethylenically unsaturated monomers. However, many of these compounds have not been fully satisfactory.

U.S. Patent No. 2,867,672 discloses that the polymerization of uninhibited styrene condensing in liquid form on the surfaces containing the vapor space above the liquid level of the main body of styrene in a tank may be minimized by spraying the surfaces enclosing the vapor space with a styrene polymerization inhibitor.

U.S. Patent No. 4,086,147 discloses a process for the distillation of readily polymerizable vinyl aromatic compounds comprising subjecting a vinyl aromatic compound to elevated temperatures in a distillation system in the presence of a polymerization inhibitor comprising m-nitro-p-cresol.

U.S. Patent No. 4,468,343 discloses a compound and a process for utilizing the compound to prevent the polymerization of vinyl aromatic compounds, such as styrene, during heating. The composition includes effective amounts of 2,6-dinitro-p-cresol and either a phenylenediamine or 4-tert-butylcatechol respectively, to act as a polymerization co-inhibitor system in the presence of oxygen.

U.S. Patent No. 4,670,131 discloses controlling the fouling of equipment used for processing of organic feed streams containing olefinic compounds by inhibiting polymerization of the olefinic compounds by carrying out the processing in the presence of from about 20 ppb to less than 1000 ppb of a stable free radical, such as a nitroxide.

U.S. Patent No. 5,254,760 discloses the inhibition of the polymerization of a vinyl aromatic compound, such as styrene, during distillation or purification by the presence of at least one stable nitroxyl compound together with at least one aromatic nitro compound.

U.S. Patent No. 5,290,888 discloses a process for stabilizing an ethylenically unsaturated monomer or oligomer from premature polymerization whereby a stabilizing amount of an N-hydroxy substituted hindered amine is added to said polymerizable monomer or oligomer. The ethylenically unsaturated monomer or oligomer encompasses vinyl monomers or oligomers bearing at least one polymerizable moiety. The N-hydroxy substituted hindered amine is said to inhibit premature polymerization in the liquid and/or vapor phase.

U.S. Patent No. 5,446,220 discloses methods for inhibiting the polymerization of vinyl aromatic monomers in oxygen-free processing systems. These methods comprise adding from 1 to about 10,000 parts per million parts monomer of a combination of a dinitrophenol compound, a hydroxylamine compound and a phenylenediamine compound. Preferably, 2-sec-butyl-4,6-dinitrophenol or 4,6-dinitro-o-cresol are used in combination with bis-(hydroxypropyl)hydroxylamine and N,N' -di-sec-butyl-p-phenylenediamine.

U.S. Patent No. 5,545,786 discloses that nitroxyl inhibitors in combination with some oxygen reduce the premature polymerization of vinyl aromatic monomers during the manufacturing processes for such monomers. It is also disclosed that even small quantities of air used in combination with the nitroxyl inhibitors result in vastly prolonged inhibition times for said monomers.

U.S. Patent No. 5,932,735 discloses that selected derivatives of 1-oxyl-2,2,6,6-tetramethyl-4-hydroxypiperidine are effective as inhibitors to prevent the premature polymerization of acrylic and methacrylic acids, their esters, their amides, vinyl acetate and acrylonitrile in the presence of water.

U.S. Patent No. 6,143,205 discloses a mixture for inhibiting the premature polymerization of monomers that contains (A) vinyl-containing monomers, and (B) an effective amount of a mixture of (i) from 0.05 to 4.5% by weight, based on the total mixture (B), of at least one N-oxyl compound of a secondary amine which carries no hydrogen atoms on the α-carbon atoms and (ii) from 99.95 to 95.5% by weight, based on the total mixture (B), of at least one nitro compound.

Russian patents 1,027,150; 1,139,722; and 1,558,888 disclose decreased polymer formation during normal operating conditions (true inhibitors), but do not protect the system in emergency feed shut off situations, i.e., there is no retarder effect.

### SUMMARY OF THE INVENTION

In accordance with the present invention, inhibiting systems comprising sulfonated phenols have been found to be excellent inhibitors and retarders to prevent polymerization of ethylenically unsaturated monomers, especially vinyl aromatic compounds, when used with nitrophenols, such as 2,4-dinitro-o-sec-butylphenol (DNBP). Optionally, this inhibitor system can be used in combination with nitroxyl radical type compounds or nitrosoanilines and amines.

It is an advantage of the present invention that the sulfonated phenols can be produced in the DNBP manufacturing process. Since DNBP is a preferred second component of the claimed inhibitor blend, both components can be manufactured in the same process. Accordingly, manufacturing can be simplified by producing both components in the same process at the same location. This provides the economic advantage that the material can be produced in an already existing process at low cost without capital investment. From a customer point of view, an economic advantage is realized owing to the low manufacturing cost (low price) and the superior performance of the claimed inhibitor blend. The latter results in low inhibitor usage and low polymer make.

It is thus an object of the present invention to develop a highly efficient and inexpensive polymerization inhibitor blend with superb true inhibitor and retarder capabilities.

This and other objects are obtained by the present invention, which is directed to a method for inhibiting the premature polymerization and the polymer growth of ethylenically unsaturated monomers comprising adding to said monomers an effective amount of a combination of
(A) at least one inhibitor that is a sulfonated phenol of the formula: 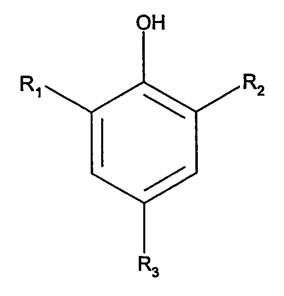 wherein
   (1) R₂ is selected from the group consisting of hydrogen and hydrocarbyl; and
   (2) R₁ and R₃ are independently selected from the group consisting of hydrogen and SO₃H, provided that at least one of R₁ and R₃ is SO₃H; and
(B) at least one inhibitor that is a nitrophenol.

In a preferred embodiment, the present invention is directed to a method for inhibiting the premature polymerization and the polymer growth of ethylenically unsaturated monomers comprising adding to said monomers an effective amount of a combination of
(A) at least one inhibitor that is a sulfonated phenol of the formula: 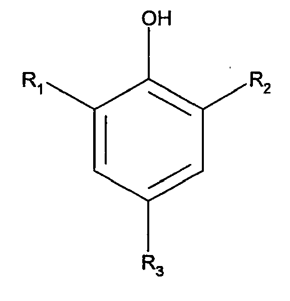 wherein
   (1) R₂ is selected from the group consisting of hydrogen and hydrocarbyl; and
   (2) R₁ and R₃ are independently selected from the group consisting of hydrogen and SO₃H, provided that at least one of R₁ and R₃ is SO₃H;
(B) at least one inhibitor that is a nitrophenol;
(C) at least one inhibitor selected from the group consisting of nitroxyl compounds and nitrosoanilines; and
(D) at least one amine.

In another aspect, the present invention is directed to a composition comprising a combination of
(A) at least one inhibitor that is a sulfonated phenol of the formula: 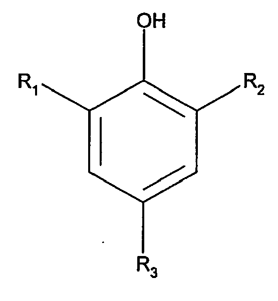 wherein
   (1) R₂ is selected from the group consisting of hydrogen and hydrocarbyl; and
   (2) R₁ and R₃ are independently selected from the group consisting of hydrogen and SO₃H, provided that at least one of R₁ and R₃ is SO₃H;
(B) at least one inhibitor that is a nitrophenol;
(C) at least one inhibitor selected from the group consisting of nitroxyl compounds and nitrosoanilines; and
(D) at least one amine.

### DESCRIPTION OF THE PREFERRED EMBODIMENTS

As stated above, the present invention is directed to inhibiting systems comprising a combination of
(A) at least one inhibitor that is a sulfonated phenol of the formula: 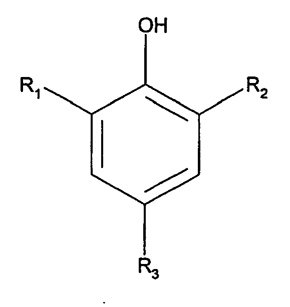 wherein
   (1) R₂ is selected from the group consisting of hydrogen and hydrocarbyl; and
   (2) R₁ and R₃ are independently selected from the group consisting of hydrogen and SO₃H, provided that at least one of R₁ and R₃ is SO₃H; and
(B) at least one inhibitor that is a nitrophenol.

In a preferred embodiment the inhibiting system further comprises at least one amine and at least one additional inhibitor selected from the group consisting of nitroxyl compounds and nitrosoanilines.

Where R₂ is hydrocarbyl, it is preferably a straight chain or branched chain alkyl or alkenyl group of from 1 to to 50 carbon atoms, more preferably of from 1 to 18 carbon atoms including, but not limited to, methyl, ethyl, propyl, butyl, pentyl, hexyl, heptyl, octyl, 2-ethyl hexyl, nonyl, decyl, undecyl, dodecyl, tridecyl, tetradecyl, pentadecyl, hexadecyl, heptadecyl, octadecyl, oleyl, nonadecyl, eicosyl, heneicosyl, docosyl, tricosyl, tetracosyl, pentacosyl, triacontyl, isomers of the foregoing (such as, for example, isopropyl, sec-butyl, neopentyl, etc.), and the like; or cyclic alkyl groups, such as cyclopentyl, cyclohexyl, cycloheptyl, cyclooctyl, and cyclododecyl.

Nitrophenols that can be employed in the practice of the present invention include, but are not limited to, 2,6-dinitro-4-methylphenol, 2-nitro-4-methylphenol, 2,4-dinitro-1-naphthol, 2,4,6-trinitrophenol (picric acid), 2,4-dinitro-6-methylphenol, 2,4-dinitrophenol, 2,4-dinitro-6-sec-butylphenol, 4-cyano-2-nitrophenol, 3-iodo-4-cyano-5-nitrophenol, *m-*nitro-*p*-cresol, 2,6-dinitro*-p*-cresol, and the like. 2,4-Dinitro-6-sec-butylphenol is preferred.

Where the inhibiting system of the present invention comprises an additional inhibitor that is a nitroxyl compound, the nitroxyl compound is preferably a stable hindered nitroxyl compound having the structural formula: 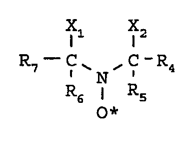 wherein R₄ and R₇ are independently selected from the group consisting of hydrogen, alkyl, and heteroatom-substituted alkyl and R₅ and R₆ are independently selected from the group consisting of alkyl and heteroatom-substituted alkyl; and X₁ and X₂(1) are independently selected from the group consisting of halogen, cyano, COOR₇, -S-COR₇, -OCOR₇, (wherein R₇ is alkyl or aryl), amido, -S-C₆H₅, carbonyl, alkenyl, or alkyl of 1 to 15 carbon atoms, or (2) taken together, form a ring structure with the nitrogen.

In a particularly preferred embodiment, the stable hindered nitroxyl compound has the structural formula: 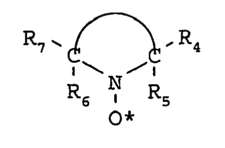 wherein R₄ and R₇ are independently selected from the group consisting of hydrogen, alkyl, and heteroatom-substituted alkyl and R₅ and R₆ are independently selected from the group consisting of alkyl and heteroatom-substituted alkyl, and the 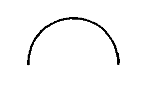 portion represents the atoms necessary to form a five-, six-, or seven-membered heterocyclic ring.

Accordingly, one of the several classes of cyclic nitroxides that can be employed in the practice of the present invention can be represented by the following structural formula: 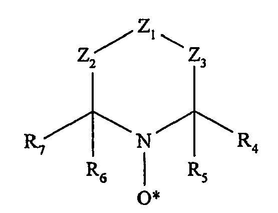 wherein Z₁, Z₂, and Z₃ are independently selected from the group consisting of oxygen, sulfur, secondary amines, tertiary amines, phosphorus of various oxidation states, and substituted or unsubstituted carbon atoms, such as >CH₂, >CHCH₃, >C=O, >C(CH₃)₂, >CHBr, >CHCl, >CHI, >CHF, >CHOH, >CHCN, >C(OH)CN, >CHCOOH, >CHCOOCH₃, >CHCOOC₂H₅, >C(OH)COOC₂H₅, >C(OH)COOCH₃, >C(OH)CHOHC₂H₅, >CR₈OR₉, >CHNR₈R₉, >CCONR₈R₉, >C=NOH, >C=CH-C₆H₅, >CF₂, >CCl₂, >CBr₂, >Cl₂, >CR₈PR₁₃R₁₄R₁₅, and the like, where R₈ and R₉ are independently selected from the group consisting of hydrogen, alkyl, aryl, and acyl and R₁₃, R₁₄, and R₁₅ are independently selected from the group consisting of unshared electrons, alkyl, aryl, =O, OR₁₆, and NR₁₇R₁₈, where R₁₆, R₁₇, and R₁₈ are independently selected from the group consisting of hydrogen, alkyl, and aryl. Where R₈ and/or R₉ are alkyl, it is preferred that they be a lower alkyl (i.e., one having one to five carbon atoms, e.g., methyl, ethyl, propyl, butyl, pentyl, and isomers thereof).

Where R₈ and/or R₉ are aryl, it is preferred that they be aryl of from 6 to 10 carbon atoms, e.g., phenyl or naphthyl, which, in addition, may be substituted with non-interfering substituents, e.g., lower alkyl groups, halogens, and the like.

Where R₈ and/or R₉ are acyl, it is preferred that they be acyl of the structure 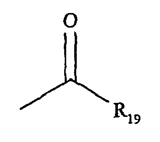 where R₁₉ is alkyl, aryl, OR₂₀, or NR₂₀R₂₁ and where R₂₀ and R₂₁ are alkyl, aryl, or 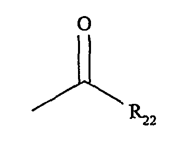 where R₂₂ is alkyl or aryl. Where R₁₉, R₂₀, R₂₁, or R₂₂ are alkyl, they are preferably alkyl of from 1 to 15 carbon atoms, more preferably lower alkyl of from 1 to 5 carbon atoms, as described above. Where R₁₉, R₂₀, R₂₁, or R₂₂ are aryl, they are preferably aryl of from 6 to 10 carbon atoms, as described above.

Another of the several classes of cyclic nitroxides that can be employed in the practice of the present invention can be represented by the following structural formula: 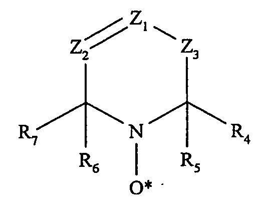 wherein Z₁ and Z₂, which may be the same or different, are nitrogen or substituted or unsubstituted carbon atoms, such as =C(H)-, =C(CH₃)-, =C(COOH)-, =C(COOCH₃)-, =C(COOC₂H₅)-, =C(OH)-, =C(CN)-, =C(NR₈R₉)-, =C(CONR₈R₉)-, and the like, and where Z₃, R₈, and R₉ are as described above.

The cyclic nitroxides employed in the practice of the present invention can also be derived from five-membered rings. These compounds are of the structure: 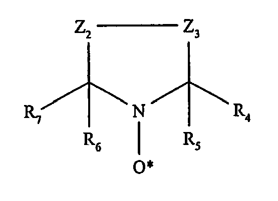 wherein Z₂ and Z₃, which may be the same or different, are sulfur, oxygen, secondary amines, tertiary amines, phosphorus of various oxidation states, or substituted or unsubstituted carbon atoms, such as, >CH₂, >CHCH₃, >C=O, >C(CH₃)₂, >CHBr, >CHCl, >CHI, >CHF, >CHOH, >CHCN, >C(OH)CN, >CHCOOH, >CHCOOCH₃, >CHCOOC₂H₅, >C(OH)COOC₂H₅, >C(OH)COOCH₃, >C(OH)CHOHC₂H₅, >CR₈OR₉, >CHNR₈R₉, >CCONR₈R₉, >C=NOH, >C=CH-C₆H₅, CF₂, CCl₂, CBr₂, CI₂, >CR₈PR₁₃R₁₄R₁₅, and the like, wherein the several R groups are as described above.

The cyclic nitroxides employed in the practice of the present invention can also have the structure: 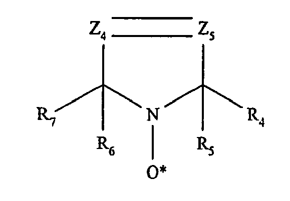 wherein Z₄ and Z₅, which can be the same or different, can be nitrogen or a substituted or unsubstituted carbon atom, such as =C(H)-, =C(CH₃)-, =C(COOH)-, =C(COOCH₃)-, =C(COOC₂H₅)-, =C(OH)-, =C(CN)-, =C(NR₈R₉)-, =C(CONR₈R₉)-, and the like, where R₈ and R₉ are as described above.

Another class of cyclic nitroxides that can be employed in the practice of the present invention is of the structure: 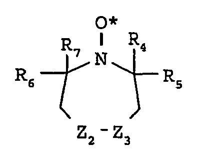 wherein Z₂ and Z₃, which may be the same or different, are sulfur, oxygen, secondary amines, tertiary amines, or substituted or unsubstituted carbon atoms, such as, >CH₂, >CHCH₃, >C=O, >C(CH₃)₂, >CHBr, >CHCl, >CHI, >CHF, >CHOH, >CHCN, >C(OH)CN, >CHCOOH, >CHCOOCH₃, >CHCOOC₂H₅, >C(OH)COOC₂H₅, >C(OH)COOCH₃, >C(OH)CHOHC₂H₅, >CHNR₈R₉, >CCONR₈R₉, >CR₈OR₉, >C=NOH, >C=CH-C₆H₅, CF₂, CCl₂, CBr₂, CI₂, >CR₈PR₁₃R₁₄R₁₅, and the like, where the several R groups are as described above.

Further, two or more nitroxyl groups can be present in the same molecule, for example, by being linked through one or more of the Z-type moieties by a linking group E, as disclosed in U.S. Patent Number 5,254,760.

As stated above, for all the nitroxyl structures above, R₄ and R₇ are independently selected from the group consisting of hydrogen, alkyl, and heteroatom-substituted alkyl and R₅ and R₆ are independently selected from the group consisting of alkyl and heteroatom-substituted alkyl. The alkyl (or heteroatom-substituted alkyl) groups R₄ through R₇ can be the same or different and preferably contain 1 to 15 carbon atoms, e.g., methyl, ethyl, propyl, butyl, pentyl, hexyl, heptyl, octyl, nonyl, decyl, undecyl, dodecyl, tridecyl, tetradecyl, pentadecyl, and the like, and isomers thereof, e.g., t-butyl, 2-ethylhexyl, and the like. It is more preferred that R₄ through R₇ be independently selected lower alkyl (or heteroatom-substituted lower alkyl) of one to five carbon atoms (e.g., methyl, ethyl, propyl, butyl, pentyl, and isomers thereof). Where heteroatom substituents are present, they can, for example, include halogen, oxygen, sulfur, nitrogen, and the like. It is most preferred that all of R₄ through R₇ be methyl.

Examples of suitable nitroxide free radical compounds that can be used in combination with the hydrogen donor or electron acceptor in the practice of the present invention, include, but are not limited to:
N,N-di-*tert*-butylnitroxide;
N,N-di-*tert*-amylnitroxide;
N-*tert*-butyl-2-methyl-1-phenyl-propylnitroxide;
N-*tert*-butyl-1-diethylphosphono-2,2-dimethylpropylnitroxide;
2,2,6,6-tetramethyl-piperidinyloxy;
4-amino-2,2,6,6-tetramethyl-piperidinyloxy;
4-hydroxy-2,2,6,6-tetramethyl-piperidinyloxy;
4-oxo-2,2,6,6-tetramethyl-piperidinyloxy;
4-dimethylamino-2,2,6,6-tetramethyl-piperidinyloxy;
4-ethanoyloxy-2,2,6,6-tetramethyl-piperidinyloxy;
2,2,5,5-tetramethylpyrrolidinyloxy;
3-amino-2,2,5,5-tetramethylpyrrolidinyloxy;
2,2,4,4-tetramethyl-1-oxa-3-azacyclopentyl-3-oxy;
2,2,4,4-tetramethyl-1-oxa-3-pyrrolinyl-1-oxy-3-carboxylic acid;
2,2,3,3,5,5,6,6-octamethyl-1,4-diazacyclohexyl-1,4-dioxy;
4-bromo-2,2,6,6-tetramethyl-piperidinyloxy;
4-chloro-2,2,6,6-tetramethyl-piperidinyloxy;
4-iodo-2,2,6,6-tetramethyl-piperidinyloxy;
4-fluoro-2,2,6,6-tetramethyl-piperidinyloxy;
4-cyano-2,2,6,6-tetramethyl-piperidinyloxy;
4-carboxy-2,2,6,6-tetramethyl-piperidinyloxy;
4-carbomethoxy-2,2,6,6-tetramethyl-piperidinyloxy;
4-carbethoxy-2,2,6,6-tetramethyl-piperidinyloxy;
4-cyano-4-hydroxy-2,2,6,6-tetramethyl-piperidinyloxy;
4-methyl-2,2,6,6-tetramethyl-piperidinyloxy;
4-carbethoxy-4-hydroxy-2,2,6,6-tetramethyl-piperidinyloxy;
4-hydroxy-4-(1-hydroxypropyl)-2,2,6,6-tetramethyl-piperidinyloxy;
4-methyl-2,2,6,6-tetramethyl-1,2,5,6-tetrahydropyridine -1-oxyl;
4-carboxy-2,2,6,6-tetramethyl-1,2,5,6-tetrahydropyridine -1-oxyl;
4-carbomethoxy-2,2,6,6-tetramethyl-1,2,5,6-tetrahydropyridine-1-oxyl;
4-carbethoxy-2,2,6,6-tetramethyl-1,2,5,6-tetrahydropyridine-1-oxyl;
4-amino-2,2,6,6-tetramethyl-1,2,5,6-tetrahydropyridine -1-oxyl;
4-amido-2,2,6,6-tetramethyl-1,2,5,6-tetrahydropyridine -1-oxyl;
3,4-diketo-2,2,5,5-tetramethylpyrrolidinyloxy;
3-keto-4-oximino-2,2,5,5-tetramethylpyrrolidinyloxy;
3-keto-4-benzylidine-2,2,5,5-tetramethylpyrrolidinyloxy;
3-keto-4,4-dibromo-2,2,5,5-tetramethylpyrrolidinyloxy;
2,2,3,3,5,5-hexamethylpyrrolidinyloxy;
3-carboximido-2,2,5,5-tetramethylpyrrolidinyloxy;
3-oximino-2,2,5,5-tetramethylpyrrolidinyloxy;
3-hydroxy-2,2,5,5-tetramethylpyrrolidinyloxy;
3-cyano-3-hydroxy-2,2,5,5-tetramethylpyrrolidinyloxy;
3-carbomethoxy-3-hydroxy-2,2,5,5-tetramethylpyrrolidinyloxy;
3-carbethoxy-3-hydroxy-2,2,5,5-tetramethylpyrrolidinyloxy;
2,2,5,5-tetramethyl-3-carboxamido-2,5-dihydropyrrole-1-oxyl;
2,2,5,5-tetramethyl-3-amino-2,5-dihydropyrrole-1-oxyl;
2,2,5,5-tetramethyl-3-carbethoxy-2,5-dihydropyrrole-1-oxyl;
2,2,5,5-tetramethyl-3-cyano-2,5-dihydropynrole-1-oxyl;
bis(1-oxyl-2,2,6,6-tetramethylpiperidin-4-yl)succinate;
bis(1-oxyl-2,2,6,6-tetramethylpiperidin-4-yl)adipate;
bis(1-oxyl-2,2,6,6-tetramethylpiperidin-4-yl)sebacate;
bis(1-oxyl-2,2, 6,6-tetramethylpiperidin-4-yl)n-butylmalonate;
bis(1-oxyl-2,2, 6,6-tetramethylpiperidin-4-yl)phthalate;
bis(1-oxyl-2,2,6,6-tetramethylpiperidin-4-yl)isophthalate;
bis(1-oxyl-2,2,6,6-tetramethylpiperidin-4-yl)terephthalate;
bis(1-oxyl-2,2,6,6-tetramethylpiperidin-4-yl)hexahydroterephthalate;
N,N'-bis(1-oxyl-2,2,6,6-tetramethylpiperidin-4-yl)adipamide;
N-(1-oxyl-2,2,6,6-tetramethylpiperidin-4-yl)-caprolactam;
N-(1-oxyl-2,2,6,6-tetramethylpiperidin-4-yl)-dodecylsuccinimide;
2,4,6-tris-[N-butyl-N-(1-oxyl-2,2,6,6-tetramethylpiperidin-4-yl)]-s-triazine;
4,4'-ethylenebis(1-oxyl-2,2,6,6-tetramethylpiperazin-3-one); and the like.

As used herein, the abbreviation TEMPO stands for 2,2,6,6-tetramethyl-1-piperidinyloxy. Thus, 4-amino-TEMPO is 4-amino-2,2,6,6-tetramethyl-1-piperidinyloxy; 4-hydroxy-TEMPO is 4-hydroxy-2,2,6,6-tetramethyl-1-piperidinyloxy (also known in the art as HTEMPO); 4-oxo-TEMPO is 4-oxo-2,2,6,6-tetramethyl-1-piperidinyloxy; and so on.

It is preferred that one member of the combination employed in the practice of the present invention be 4-amino-TEMPO, 4-oxo-TEMPO, 4-hydroxy-TEMPO, or TEMPO.

Blends of two or more of the foregoing, e.g., 4-amino-TEMPO and 4-oxo-TEMPO, can also be employed.

Such stable nitroxide free radical compounds can be prepared by known methods. (See, for example, U.S. Patent Numbers 3,163,677; 3,334,103; 3,372,182; 3,422,144; 3,494,930; 3,502,692; 3,873,564; 3,966,711; and 4,665,185.) They are suitable for use over a wide range of temperatures, but distillation temperatures employed with the ethylenically unsaturated monomers that are stabilized by the process of the present invention typically range from about 60°C to about 180°C, preferably from about 70°C to about 165°C, and, more preferably, from about 80°C to about 150°C. Such distillations are generally performed at an absolute pressure in the range of 1330 Pa to 16000 Pa (about 10 to about 1,200 mm of Hg).

Where the inhibiting system of the present invention comprises an additional inhibitor that is a nitrosoaniline, it can be an N-nitrosoaniline or a C-nitrosoaniline. Preferably, the nitrosoaniline compound is a C-nitrosoaniline.

C-nitrosoaniline compounds can be prepared by C-nitrosation of the corresponding anilines in any typical manner used for the C-nitrosation of aromatic amines. For example, reaction of the amine with cold nitrous acid produces an N-nitroso compound that rearranges to a para-nitrosoaniline under the influence of an excess of hydrochloric acid. In some cases, it is more convenient to effect the nitrosation and rearrangement in one step by conducting the reaction in methanol solution in the presence of an excess of hydrogen chloride under anhydrous conditions. This procedure is described in U.S. Patent Number 2,046,356.

Those skilled in the art will be aware that nitrosoaniline derivatives are understood to tautomerize to quinone imine oxime derivatives, i.e., 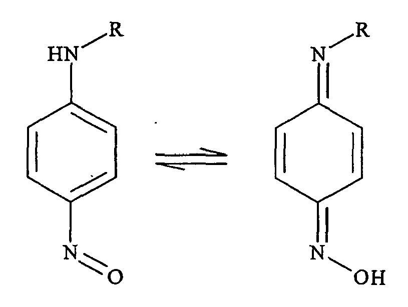 See, for example, Sidgwick, N.V., *The Organic Chemistry of Nitrogen,* Third Edition, Clarendon Press, Oxford, 1966. Thus, both forms can be present, especially in solution at elevated temperatures, and can be expected to contribute to the inhibiting activity of these compounds.

The nitrosoanilines that can be employed in the practice of the present invention are preferably of the structure: 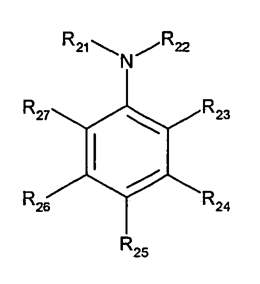 wherein
R₂₁ and R₂₂ are independently selected from the group consisting of hydrogen, alkyl, aryl, acyl, hydroxyl, alkoxy, nitroso, and sulfonyl, or R₂₁ and R₂₂ can form a cyclic ring that is aryl, cycloalkyl, polyaryl, or heterocyclic;
R₂₃ through R₂₇ are independently selected from the group consisting of hydrogen, alkyl, aryl, acyl, hydroxyl, alkoxy, acyloxy, NR₂₈(R₂₉), nitro, nitroso, halogen, and sulfonyl, or any two adjacent R's can form a cyclic ring that is aryl, cycloalkyl, polyaryl, or heterocyclic, provided that at least one of R₂₃ through R₂₇ must be a nitroso group; and
R₂₈ and R₂₉ are independently selected from the group consisting of hydrogen, alkyl, aryl, acyl, and nitroso. Preferably R₂₈ is hydrogen and R₂₉ is alkyl.

Where the inhibiting system of the present invention comprises an additional inhibitor that is an amine, the amine can be a primary, secondary, or tertiary amine, and can comprise alkyl groups, aryl groups, or combinations thereof. Such amines include, but are not limited to, α-naphthylamine, thiodiarylamines, *p-*phenylenediamine, *o*-phenylenediamine, 2,4-diamino diphenylamine, cyclohexyl naphthyl amine, polybutyl amines, methyl aniline, diphenyl-*p-*phenylene diamine, phenyl-β-naphthylamine, isopropoxydiphenylamine, aldol-α-naphthyl amine, symmetrical di-β-naphthyl-*p-*phenylenediamine, trimethyl dihydroquinoline, ditolylamines, phenyl-α-naphthylamine, phenyl-β-naphthylamine, diaminophenol, 4-cyclohexylaminophenol, p-aminophenol, *o-*aminophenol, 5-amino-2-hydroxytoluene, and the like.

The ethylenically unsaturated monomer, the premature polymerization and polymer growth of which is an object of the present invention, can be any such monomer for which unintended polymerization and/or polymer growth during its manufacture, storage, and/or distribution is a problem. Among those monomers that will benefit from the practice of the present invention are: styrene, α-methylstyrene, styrene sulfonic acid, vinyltoluene, divinylbenzenes, polyvinylbenzenes, alkylated styrene, 2-vinylpyridine, acrylonitrile, methacrylonitrile, methyl acrylate, ethyl acrylate, methyl methacrylate, ethyl methacrylate, acrylic acid, methacrylic acid, butadiene, chloroprene, isoprene, and the like.

The ethylenically unsaturated monomers will not necessarily be stabilized indefinitely by the presence of the inhibitor(s), especially when the monomers are heated as in distillation, but they can be considered to be stabilized as long as A) there is a measurable increase in the time for which they can be heated before the onset of polymerization and/or polymer growth in a static system, B) the amount of polymer made at a constant temperature remains constant over time in a dynamic system, and/or C) the rate of polymer growth is significantly slower than when the growth inhibiting system is not present.

Those skilled in the art will understand that, if desired, free radical scavengers can also be included in the practice of the present invention. For example, air or O₂, as disclosed in U.S. Patent Numbers 5,545,782 and 5,545,786, can be added, as can the aromatic nitro compounds disclosed in U.S. Patent Number 5,254,760, the dihetero-substituted benzene compounds having at least one transferable hydrogen, e.g., a quinone derivative such as the mono-methyl-ether of hydroquinone disclosed in European Patent Application 0 765 856 A1, the iron compounds disclosed in WO 98/25872, and other inhibitors, e.g., phenolics and certain inorganic salts, well-known to those skilled in the art.

The polymerization inhibitors can be introduced into the monomer to be protected by any conventional method. They can, for example, be added as a concentrated solution in suitable solvents just upstream from the point of desired application by any suitable means. In addition, individual inhibiting components can be injected separately into the distillation train along with the incoming feed and/or through separate and multiple entry points, provided there is an efficient distribution of the inhibiting composition. Since the inhibitors are gradually depleted during the distillation operation, it is generally advantageous to maintain the appropriate amount of them in the distillation apparatus by adding them during the course of the distillation process. Adding inhibitors can be done either on a generally continuous basis or intermittently, in order to maintain the inhibitor concentration above the minimum required level.

The total inhibitor concentration should be from about 1 to about 2000 ppm versus the monomer being inhibited; preferably from about 5 to about 1000 ppm, depending on the conditions of use.

The ratio of the first component (A) to the second component (B), based on the total of both components is from about 1 to 100 wt % A : about 99 to 0 wt % B; preferably, about 25-75 wt % A : about 75-25 wt% B; more preferably about 50-75 wt % A : about 50-25 wt % B.

The advantages and the important features of the present invention will be more apparent from the following examples.

### EXAMPLES

### Example 1

Concentrated H₂SO₄ (280 grams, 2.8 moles) was placed in a one liter flask equipped with a mechanical stirrer, thermocouple, heating mantle, condenser, and plastic tube. The acid was preheated to 40°C and 300 grams of o-sec-butylphenol (OSBP) (2 moles) was loaded through the plastic tube fast enough to heat the system to 82°C. The initial temperature of 40°C reached 82°C after 40 minutes. After that, the reaction mixture had to be heated to maintain the temperature at 82°C. The addition took 1 hour and 45 minutes. The product, sulfonated OSBP (SOSBP), was used for inhibitor performance tests in the presence of DNBP.

### Example 2

The styrene inhibitor and retarder properties of this material were tested in a Continuous Dynamic Reboiler Test monitoring the polymer formation with UV spectrophotometry. According to this test, the inhibitor is added to styrene monomer from which *tert*-butylcatechol (TBC) is previously removed by distillation. This styrene (180 grams) is loaded into a flask, which is immersed into an oil bath. The temperature of styrene is usually 116°C. During the test, a fresh feed is charged into the flask at the rate of three grams/minute and, at the same time, the material from flask is discharged at the same rate. The steady stage is continued until equilibrium. For feed shut off stage, the charging and discharging are discontinued. Samples are taken every hour at the steady stage and every 5-10 minutes at feed shut off.

After 5 hours of steady stage, at 50 ppm/100 ppm SOSBP/DNBP concentration, 0.0007 % polymer was measured while 1.5 hour feed shut off resulted in 0.024 % polymer.

### Example 3

Continuous Dynamic Reboiler Test of SOSBP/NMPlDNBP at a concentration of 250 ppm/285 ppm/250 ppm resulted in 0.0039 polymer in steady stage and 0.25% polymer after two hours feed shut off. NMP (1-methyl-2-pyrrolidinone) was added to neutralize the acidic SOSBP.

### Example 4

Continuous Dynamic Reboiler Test of SOSBP/DNBP at a concentration of 250 ppm/250 ppm resulted in 0.0004 % polymer in 5 hours steady stage and 0.025 % polymer after 2 hours feed shut off.

### Example 5

Continuous Dynamic Reboiler Test ofN,N-diethyl-4-nitrosoaniline/SOSBP/NMP/DNBP at a concentration of 100 ppm/250 ppm/170 ppm/250 ppm resulted in 0.0038 % polymer in 5 hours steady stage and 0.315 % polymer after 2 hours feed shut off.

### Example 6

Continuous Dynamic Reboiler Test of 4-oxo-TEMPO/SOSBP/NMP/DNBP at a concentration of 100 ppm/250 ppm/187 ppm/250 ppm resulted in 0.0004 % polymer in 5 hours steady stage and 0.016 % polymer after 2 hours feed shut off.

### Example 7

Continuous Dynamic Reboiler Test of DNBP alone at 500 ppm concentration revealed 0.11 % of polymer in steady stage and 1.18 % of polymer after 2 hours feed shut off.

## Claims

1. A method for inhibiting the premature polymerization and the polymer growth of ethylenically unsaturated monomers comprising adding to said monomers an effective amount of a combination of
(A) at least one inhibitor that is a sulfonated phenol of the formula: 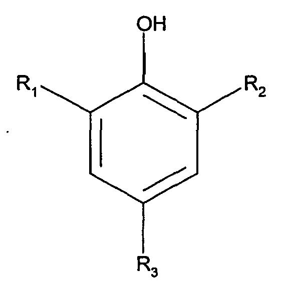 wherein
(1) R₂ is selected from the group consisting of hydrogen and hydrocarbyl; and
(2) R₁ and R₃ are independently selected from the group consisting of hydrogen and SO₃H, provided that at least one of R₁ and R₃ is SO₃H; and
(B) at least one inhibitor that is a nitrophenol.

2. The method of claim 1 wherein R₂ is a straight chain or branched chain alkyl or alkenyl group of from 1 to 50 carbon atoms.

3. The method of claim 1 wherein R₂ is selected from the group consisting of methyl, ethyl, propyl, butyl, pentyl, hexyl, heptyl, octyl, 2-ethyl hexyl, nonyl, decyl, undecyl, dodecyl, tridecyl, tetradecyl, pentadecyl, hexadecyl, heptadecyl, octadecyl, oleyl, nonadecyl, eicosyl, heneicosyl, docosyl, tricosyl, tetracosyl, pentacosyl, triacontyl, isomers of the foregoing, cyclopentyl, cyclohexyl, cycloheptyl, cyclooctyl, and cyclododecyl.

4. The method of claim 3 wherein R₂ is *sec*-butyl.

5. The method of claim 1 wherein the nitrophenol is selected from the group consisting of 2,6-dinitro-4-methylphenol, 2-nitro-4-methylphenol, 2,4-dinitro-1-naphthol, 2,4,6-trinitrophenol (picric acid), 2,4-dinitro-6-methylphenol, 2,4-dinitrophenol, 2,4-dinitro-6-sec-butylphenol, 4-cyano-2-nitrophenol, 3-iodo-4-cyano-5-nitrophenol, *m-*nitro-*p-*cresol, and 2,6-dinitro-*p-*cresol

6. A method according to claim 1 for inhibiting the premature polymerization and the polymer growth of ethylenically unsaturated monomers comprising adding to said monomers an effective amount of a combination of
(A) at least one inhibitor that is a sulfonated phenol of the formula: 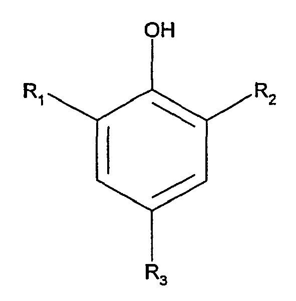 wherein
(1) R₂ is selected from the group consisting of hydrogen and hydrocarbyl; and
(2) R₁ and R₃ are independently selected from the group consisting of hydrogen and SO₃H, provided that at least one of R₁ and R₃ is SO₃H;
(B) at least one inhibitor that is a nitrophenol;
(C) at least one inhibitor selected from the group consisting of nitroxyl compounds and nitrosoanilines; and
(D) at least one amine.

7. The method of claim 6 wherein R₂ is a straight chain or branched chain alkyl or alkenyl group of from 1 to 50 carbon atoms.

8. The method of claim 6 wherein R₂ is selected from the group consisting of methyl, ethyl, propyl, butyl, pentyl, hexyl, heptyl, octyl, 2-ethyl hexyl, nonyl, decyl, undecyl, dodecyl, tridecyl, tetradecyl, pentadecyl, hexadecyl, heptadecyl, octadecyl, oleyl, nonadecyl, eicosyl, heneicosyl, docosyl, tricosyl, tetracosyl, pentacosyl, triacontyl, isomers of the foregoing, cyclopentyl, cyclohexyl, cycloheptyl, cyclooctyl, and cyclododecyl.

9. The method of claim 8 wherein R₂ is *sec*-butyl.

10. The method of claim 6 wherein the nitrophenol is selected from the group consisting of 2,6-dinitro-4-methylphenol, 2-nitro-4-methylphenol, 2,4-dinitro-1-naphthol, 2,4,6-trinitrophenol (picric acid), 2,4-dinitro-6-methylphenol, 2,4-dinitrophenol, 2,4-dinitro-6-sec-butylphenol, 4-cyano-2-nitrophenol, 3-iodo-4-cyano-5-nitrophenol, *m-*nitro-*p-*cresol, and 2,6-dinitro-*p-*cresol

11. The method of claim 6 wherein (C) is a stable hindered nitroxyl compound having the structural formula: 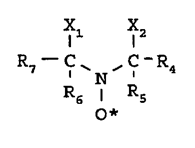 wherein R₄ and R₇ are independently selected from the group consisting of hydrogen, alkyl, and heteroatom-substituted alkyl and R₅ and R₆ are independently selected from the group consisting of alkyl and heteroatom-substituted alkyl; and X₁ and X₂ (1) are independently selected from the group consisting of halogen, cyano, COOR₇, -S-COR₇, -OCOR₇, (wherein R₇ is alkyl or aryl), amido, -S-C₆H₅, carbonyl, alkenyl, or alkyl of 1 to 15 carbon atoms, or (2) taken together, form a ring structure with the nitrogen.

12. The method of claim 6 wherein (C) is a nitrosoaniline of the structure: 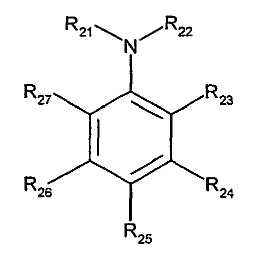 wherein
R₂₁ and R₂₂ are independently selected from the group consisting of hydrogen, alkyl, aryl, acyl, hydroxyl, alkoxy, nitroso, and sulfonyl, or R₂₁ and R₂₂ can form a cyclic ring that is aryl, cycloalkyl, polyaryl, or heterocyclic;
R₂₃ through R₂₇ are independently selected from the group consisting of hydrogen, alkyl, aryl, acyl, hydroxyl, alkoxy, acyloxy, NR₂₈(R₂₉), nitro, nitroso, halogen, and sulfonyl, or any two adjacent R's can form a cyclic ring that is aryl, cycloalkyl, polyaryl, or heterocyclic, provided that at least one of R₂₃ through R₂₇ must be a nitroso group; and
R₂₈ and R₂₉ are independently selected from the group consisting of hydrogen, alkyl, aryl, acyl, and nitroso.

13. The method of claim 6 wherein (D) is selected from the group consisting of α-naphthylamine, thiodiarylamines, *p-*phenylenediamine, *o-*phenylenediamine, 2,4-diamino diphenylamine, cyclohexyl naphthyl amine, polybutyl amines, methyl aniline, diphenyl-*p-*phenylene diamine, phenyl-β-naphthylamine, isopropoxydiphenylamine, aldol-α-naphthyl amine, symmetrical di-β-naphthyl-*p-*phenylenediamine, trimethyl dihydroquinoline, ditolylamines, phenyl-α-naphthylamine, phenyl-β-naphthylamine, diaminophenol, 4-cyclohexylaminophenol, *p-*aminophenol, *o-*aminophenol, and 5-amino-2-hydroxytoluene.

14. A composition comprising a combination of
(A) at least one inhibitor that is a sulfonated phenol of the formula: 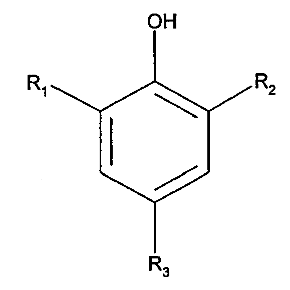 wherein
(1) R₂ is selected from the group consisting of hydrogen and hydrocarbyl; and
(2) R₁ and R₃ are independently selected from the group consisting of hydrogen and SO₃H, provided that at least one of R₁ and R₃ is SO₃H;
(B) at least one inhibitor that is a nitrophenol;
(C) an inhibitor selected from the group consisting of nitroxyl compounds and nitrosoanilines; and
(D) an amine.

15. The composition of claim 14 wherein R₂ is a straight chain or branched chain alkyl or alkenyl group of from 1 to 50 carbon atoms.

16. The composition of claim 14 wherein R₂ is selected from the group consisting of methyl, ethyl, propyl, butyl, pentyl, hexyl, heptyl, octyl, 2-ethyl hexyl, nonyl, decyl, undecyl, dodecyl, tridecyl, tetradecyl, pentadecyl, hexadecyl, heptadecyl, octadecyl, oleyl, nonadecyl, eicosyl, heneicosyl, docosyl, tricosyl, tetracosyl, pentacosyl, triacontyl, isomers of the foregoing, cyclopentyl, cyclohexyl, cycloheptyl, cyclooctyl, and cyclododecyl.

17. The composition of claim 14 wherein the nitrophenol is selected from the group consisting of 2,6-dinitro-4-methylphenol, 2-nitro-4-methylphenol, 2,4-dinitro-1-naphthol, 2,4,6-trinitrophenol (picric acid), 2,4-dinitro-6-methylphenol, 2,4-dinitrophenol, 2,4-dinitro-6-sec-butylphenol, 4-cyano-2-nitrophenol, 3-iodo-4-cyano-5-nitrophenol, *m-*nitro *p-*cresol, and 2,6-dinitro-*p-*cresol

18. The composition of claim 14 wherein (C) is a stable hindered nitroxyl compound having the structural formula: 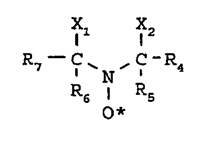 wherein R₄ and R₇ are independently selected from the group consisting of hydrogen, alkyl, and heteroatom-substituted alkyl and R₅ and R₆ are independently selected from the group consisting of alkyl and heteroatom-substituted alkyl; and X₁ and X₂(1) are independently selected from the group consisting of halogen, cyano, COOR₇, -S-COR₇, -OCOR₇, (wherein R₇ is alkyl or aryl), amido, -S-C₆H₅, carbonyl, alkenyl, or alkyl of 1 to 15 carbon atoms, or (2) taken together, form a ring structure with the nitrogen.

19. The composition of claim 14 wherein (C) is a nitrosoaniline of the structure: 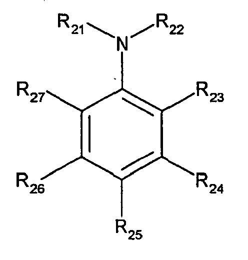 wherein
R₂₁ and R₂₂ are independently selected from the group consisting of hydrogen, alkyl, aryl, acyl, hydroxyl, alkoxy, nitroso, and sulfonyl, or R₂₁ and R₂₂ can form a cyclic ring that is aryl, cycloalkyl, polyaryl, or heterocyclic;
R₂₃ through R₂₇ are independently selected from the group consisting of hydrogen, alkyl, aryl, acyl, hydroxyl, alkoxy, acyloxy, NR₂₈(R₂₉), nitro, nitroso, halogen, and sulfonyl, or any two adjacent R's can form a cyclic ring that is aryl, cycloalkyl, polyaryl, or heterocyclic, provided that at least one of R₂₃ through R₂₇ must be a nitroso group; and
R₂₈ and R₂₉ are independently selected from the group consisting of hydrogen, alkyl, aryl, acyl, and nitroso.

20. The composition of claim 14 wherein (D) is selected from the group consisting of α-naphthylamine, thiodiarylamines, *p-*phenylenediamine, *o-*phenylenediamine, 2,4-diamino diphenylamine, cyclohexyl naphthyl amine, polybutyl amines, methyl aniline, diphenyl-*p-*phenylene diamine, phenyl-β-naphthylamine, isopropoxydiphenylamine, aldol-α-naphthyl amine, symmetrical di-β-naphthyl-*p-*phenylenediamine, trimethyl dihydroquinoline, ditolylamines, phenyl-α-naphthylamine, phenyl-β-naphthylamine, diaminophenol, 4-cyclohexylaminophenol, *p*-aminophenol, *oi*aminophenol, and 5-amino-2-hydroxytoluene.

## Patentansprüche

1. Verfahren zur Hemmung der vorzeitigen Polymerisation und des Polymerwachstums von ethylenisch ungesättigten Monomeren durch Zugeben einer wirksamen Menge einer Kombination aus
(A) mindestens einem Inhibitor, bei dem es sich um ein sulfoniertes Phenol der Formel 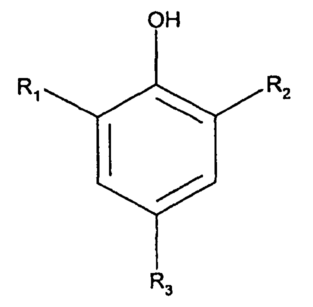 handelt, worin
(1) R₂ aus der Gruppe ausgewählt ist, die aus Wasserstoff und Kohlenwasserstoffresten besteht; und
(2) R₁ und R₃ unabhängig voneinander aus der Gruppe ausgewählt sind, die aus Wasserstoff und SO₃H besteht, wobei gilt, dass mindestens einer der Reste R₁ und R₃ für SO₃H steht;
(B) mindestens einem Inhibitor, bei dem es sich um ein Nitrophenol handelt,
zu den Monomeren.

2. Verfahren nach Anspruch 1, worin R₂ für eine geradkettige oder verzweigte Alkyl- oder Alkenylgruppe mit 1 bis 50 Kohlenstoffatomen steht.

3. Verfahren nach Anspruch 1, worin R₂ aus der Gruppe ausgewählt ist, die aus Methyl, Ethyl, Propyl, Butyl, Pentyl, Hexyl, Heptyl, Octyl, 2-Ethylhexyl, Nonyl, Decyl, Undecyl, Dodecyl, Tridecyl, Tetradecyl, Pentadecyl, Hexadecyl, Heptadecyl, Octadecyl, Oleyl, Nonadecyl, Eicosyl, Heneicosyl, Docosyl, Tricosyl, Tetracosyl, Pentacosyl, Triacontyl, Isomeren der Vorgenannten, Cyclopentyl, Cyclohexyl, Cycloheptyl, Cyclooctyl und Cyclododecyl besteht.

4. Verfahren nach Anspruch 3, worin R₂ für sek.-Butyl steht.

5. Verfahren nach Anspruch 1, worin das Nitrophenol aus der Gruppe ausgewählt ist, die aus 2,6-Dinitro-4-methylphenol, 2-Nitro-4-methylphenol, 2,4-Dinitro-1-naphthol, 2,4,6-Trinitrophenol (Picrinsäure), 2,4-Dinitro-6-methylphenol, 2,4-Dinitrophenol, 2,4-Dinitro-6-sek.-butylphenol, 4-Cyano-2-nitrophenol, 3-lod-4-cyano-5-nitrophenol, m-Nitro-p-cresol und 2,6-Dinitro-p-cresol besteht.

6. Verfahren nach Anspruch 1 zur Hemmung der vorzeitigen Polymerisation und des Polymerwachstums von ethylenisch ungesättigten Monomeren durch Zugeben einer wirksamen Menge einer Kombination aus
(A) mindestens einem Inhibitor, bei dem es sich um ein sulfoniertes Phenol der Formel 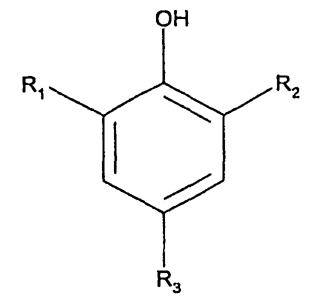 handelt, worin
(1) R₂ aus der Gruppe ausgewählt ist, die aus Wasserstoff und Kohlenwasserstoffresten besteht, und
(2) R₁ und R₃ unabhängig voneinander aus der Gruppe ausgewählt sind, die aus Wasserstoff und SO₃H besteht, wobei gilt, dass mindestens einer der Reste von R₁ und R3 für SO₃H steht;
(B) mindestens einem Inhibitor, bei dem es sich um ein Nitrophenol handelt,
(C) mindestens einem Inhibitor, der aus der Gruppe ausgewählt ist, die aus Nitroxylverbindungen und Nitrosoanilinen besteht; und
(D) mindestens einem Amin
zu den Monomeren.

7. Verfahren nach Anspruch 6, worin R₂ für eine geradkettige oder verzweigte Alkyl- oder Alkenylgruppe mit 1 bis 50 Kohlenstoffatomen steht.

8. Verfahren nach Anspruch 6, worin R₂ aus der Gruppe ausgewählt ist, die aus Methyl, Ethyl, Propyl, Butyl, Pentyl, Hexyl, Heptyl, Octyl, 2-Ethylhexyl, Nonyl, Decyl, Undecyl, Dodecyl, Tridecyl, Tetradecyl, Pentadecyl, Hexadecyl, Heptadecyl, Octadecyl, Oleyl, Nonadecyl, Eicosyl, Heneicosyl, Docosyl, Tricosyl, Tetracosyl, Pentacosyl, Triacontyl, Isomeren der Vorgenannten, Cyclopentyl, Cyclohexyl, Cycloheptyl, Cyclooctyl und Cyclododecyl besteht.

9. Verfahren nach Anspruch 8, worin R₂ für sek.-Butyl steht.

10. Verfahren nach Anspruch 6, worin das Nitrophenol aus der Gruppe ausgewählt ist, die aus 2,6-Dinitro-4-methylphenol, 2-Nitro-4-methylphenol, 2,4-Dinitro-1-naphthol, 2,4,6-Trinitrophenol (Picrinsäure), 2,4-Dinitro-6-methylphenol, 2,4-Dinitrophenol, 2,4-Dinitro-6-sek.-butylphenol, 4-Cyano-2-nitrophenol, 3-Iod-4-cyano-5-nitrophenol, m-Nitro-p-cresol und 2,6-Dinitro-p-cresol besteht.

11. Verfahren nach Anspruch 6, worin (C) eine stabile gehinderte Nitroxylverbindung der Strukturformel 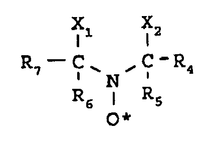 ist, worin R₄ und R₇ unabhängig voneinander aus der Gruppe ausgewählt sind, die aus Wasserstoff, Alkyl und Heteroatom-substituiertem Alkyl besteht, und R₅ und R₆ unabhängig voneinander aus der Gruppe ausgewählt sind, die aus Alkyl und Heteroatom-substituiertem Alkyl besteht; und X₁ und X₂ (1) unabhängig voneinander aus der Gruppe ausgewählt sind, die aus Halogen, Cyano, COOR₇, -S-COR₇, -OCOR₇ (worin R₇ für Alkyl oder Aryl steht), Amido, -S-C₆H₅, Carbonyl, Alkenyl oder Alkyl mit 1 bis 15 Kohlenstoffatomen besteht, oder (2) zusammengenommen eine Ringstruktur mit dem Stickstoff bilden.

12. Verfahren nach Anspruch 6, worin (C) ein Nitrosoanilin der Struktur 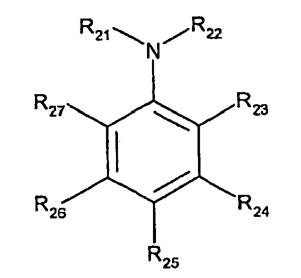 ist, worin R₂₁ und R₂₂ unabhängig voneinander aus der Gruppe ausgewählt sind, die aus Wasserstoff, Alkyl, Aryl, Acyl, Hydroxyl, Alkoxy, Nitroso und Sulfonyl besteht, oder R₂₁ und R₂₂ einen cyclischen Ring bilden können, bei dem es sich um einen Arylring, Cycloalkylring, Polyarylring oder heterocyclischen Ring handelt;
R₂₃ bis R₂₇ unabhängig voneinander aus der Gruppe ausgewählt sind, die aus Wasserstoff, Alkyl, Aryl, Acyl, Hydroxyl, Alkoxy, Acyloxy, NR₂₈(R₂₉), Nitro, Nitroso, Halogen und Sulfonyl besteht, oder beliebige zwei benachbarte Reste einen cyclischen Ring bilden können, der ein Arylring, Cycloalkylring, Polyarylring oder heterocyclischer Ring ist, vorausgesetzt, dass mindestens einer der Reste von R₂₃ bis R₂₇ eine Nitrosogruppe sein muss; und
R₂₈ und R₂₉ unabhängig voneinander aus der Gruppe ausgewählt sind, die aus Wasserstoff, Alkyl, Aryl, Acyl und Nitroso besteht.

13. Verfahren nach Anspruch 6, worin (D) aus der Gruppe ausgewählt ist, die aus α-Naphthylamin, Thiodiarylaminen, p-Phenylendiamin, o-Phenylendiamin, 2,4-Diaminodiphenylamin, Cyclohexylnaphthylamin, Polybutylaminen, Methylanilin, Diphenyl-p-phenylendiamin, Phenyl-β-naphthylamin, Isopropoxydiphenylamin, Aldol-α-naphthylamin, symmetrischem Di-β-naphthyl-p-phenylendiamin, Trimethyldihydrochinolin, Ditolylaminen, Phenyl-α-naphthylamin, Phenyl-β-naphthylamin, Diaminophenol, 4-Cyclohexylaminophenol, p-Aminophenol, o-Aminophenol und 5-Amino-2-hydroxytoluol besteht.

14. Zusammensetzung, die eine Kombination aus
(A) mindestens einem Inhibitor, der ein sulfoniertes Phenol der Formel 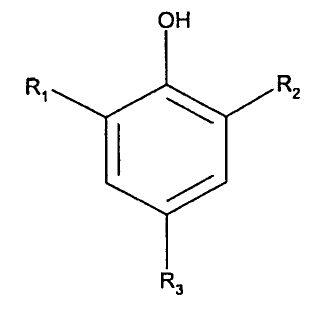 ist, worin
(1) R₂ aus der Gruppe ausgewählt ist, die aus Wasserstoff und Kohlenwasserstoffresten besteht; und
(2) R₁ und R₃ unabhängig voneinander aus der Gruppe ausgewählt sind, die aus Wasserstoff und SO₃H besteht, wobei gilt, dass mindestens einer der Reste von R₁ und R₃ für SO₃H steht;
(B) mindestens einem Inhibitor, bei dem sich um ein Nitrophenol handelt,
(C) einem Inhibitor, der aus der Gruppe ausgewählt ist, die aus Nitroxylverbindungen und Nitrosoanilinen besteht, und
(D) einem Amin
umfasst.

15. Zusammensetzung nach Anspruch 14, worin R₂ für eine geradkettige oder verzweigte Alkyl- oder Alkenylgruppe mit 1 bis 50 Kohlenstoffatomen steht.

16. Zusammensetzung nach Anspruch 14, worin R₂ aus der Gruppe ausgewählt ist, die aus Methyl, Ethyl, Propyl, Butyl, Pentyl, Hexyl, Heptyl, Octyl, 2-Ethylhexyl, Nonyl, Decyl, Undecyl, Dodecyl, Tridecyl, Tetradecyl, Pentadecyl, Hexadecyl, Heptadecyl, Octadecyl, Oleyl, Nonadecyl, Eicosyl, Heneicosyl, Docosyl, Tricosyl, Tetracosyl, Pentacosyl, Triacontyl, Isomeren der Vorgenannten, Cyclopentyl, Cyclohexyl, Cycloheptyl, Cyclooctyl und Cyclododecyl besteht.

17. Zusammensetzung nach Anspruch 14, worin das Nitrophenol aus der Gruppe ausgewählt ist, die aus 2,6-Dinitro-4-methylphenol, 2-Nitro-4-methylphenol, 2,4-Dinitro-1-naphthol, 2,4,6-Trinitrophenol (Picrinsäure), 2,4-Dinitro-6-methylphenol, 2,4-Dinitrophenol, 2,4-Dinitro-6-sek.-butylphenol, 4-Cyano-2-nitrophenol, 3-lod-4-cyano-5-nitrophenol, m-Nitro-p-cresol und 2,6-Dinitro-p-cresol besteht.

18. Zusammensetzung nach Anspruch 14, worin (C) für eine stabile gehinderte Nitroxylverbindung der Strukturformel 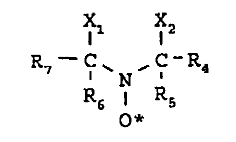 steht, worin R₄ und R₇ unabhängig voneinander aus der Gruppe ausgewählt sind, die aus Wasserstoff, Alkyl und Heteroatom-substituiertem Alkyl besteht, und R₅ und R₆ unabhängig voneinander aus der Gruppe ausgewählt sind, die aus Alkyl und Heteroatom-substituiertem Alkyl besteht, und X₁ und X₂ (1) unabhängig voneinander aus der Gruppe ausgewählt sind, die aus Halogen, Cyano, COOR₇, -S-COR₇, -OCOR₇ (worin R₇ für Alkyl oder Aryl steht), Amido, -S-C₆H₅, Carbonyl, Alkenyl oder Alkyl mit 1 bis 15 Kohlenstoffatomen besteht, oder (2) zusammengenommen eine Ringstruktur mit dem Stickstoff bilden.

19. Zusammensetzung nach Anspruch 14, worin (C) ein Nitrosoanilin der Struktur 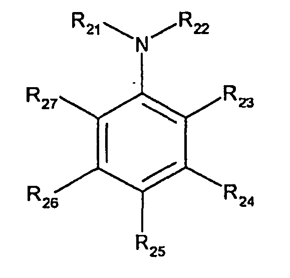 ist, worin
R₂₁ und R₂₂ unabhängig voneinander aus der Gruppe ausgewählt sind, die aus Wasserstoff, Alkyl, Aryl, Acyl, Hydroxyl, Alkoxy, Nitroso und Sulfonyl besteht, oder R₂₁, und R₂₂ einen cyclischen Ring bilden können, bei dem es sich um einen Arylring, Cycloalkylring, Polyarylring oder heterocyclischen Ring handelt;
R₂₃ bis R₂₇ unabhängig voneinander aus der Gruppe ausgewählt sind, die aus Wasserstoff, Alkyl, Aryl, Acyl, Hydroxyl, Alkoxy, Acyloxy, NR₂₈(R₂₉), Nitro, Nitroso, Halogen und Sulfonyl besteht, oder zwei benachbarte Reste einen cyclischen Ring bilden können, der ein Arylring, Cycloalkylring, Polyarylring oder heterocyclischer Ring ist, vorausgesetzt, dass mindestens einer der Reste von R₂₃ bis R₂₇ eine Nitrosogruppe sein muss; und
R₂₈ und R₂₉ unabhängig voneinander aus der Gruppe ausgewählt sind, die aus Wasserstoff, Alkyl, Aryl, Acyl und Nitroso besteht.

20. Zusammensetzung nach Anspruch 14, worin (D) aus der Gruppe ausgewählt ist, die aus α-Naphthylamin, Thiodiarylaminen, p-Phenylendiamin, o-Phenylendiamin, 2,4-Diaminodiphenylamin, Cyclohexylnaphthylamin, Polybutylaminen, Methylanilin, Diphenyl-p-phenylendiamin, Phenyl-β-naphthylamin, Isopropoxydiphenylamin, Aldol-α-napththylamin, symmetrischem Di-βnaphthyl-p-phenylendiamin, Trimethyldihydrochinolin, Ditolylaminen, Phenyl-α-naphthylamin, Phenyl-β-naphthylamin, Diaminophenol, 4-Cyclohexylaminophenol, p-Aminophenol, o-Aminophenol und 5-Amino-2-hydroxytoluol besteht.

## Revendications

1. Procédé pour inhiber la polymérisation prématurée et la croissance de polymères de monomères à insaturation éthylénique, comprenant l'addition auxdits monomères d'une quantité efficace d'une combinaison de
(A) au moins un inhibiteur qui est un phénol sulfoné de formule : 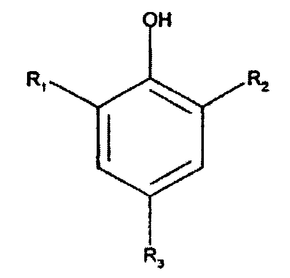 dans laquelle
(1) R₂ est choisi dans le groupe constitué de l'hydrogène et d'un groupe hydrocarbyle ; et
(2) R₁ et R₃ sont indépendamment choisis dans le groupe constitué de l'hydrogène et de SO₃H, sous réserve qu'au moins l'un de R₁ et de R₃ soit SO₃H ; et
(B) au moins un inhibiteur qui est un nitrophénol.

2. Procédé selon la revendication 1, dans lequel R₂ est un groupe alkyle à chaîne linéaire ou à chaîne ramifiée ou un groupe alcényle ayant de 1 à 50 atomes de carbone.

3. Procédé selon la revendication 1, dans lequel R₂ est choisi dans le groupe constitué des groupes méthyle, éthyle, propyle, butyle, pentyle, hexyle, heptyle, octyle, 2-éthylhexyle, nonyle, décyle, undécyle, dodécyle, tridécyle, tétradécyle, pentadécyle, hexadécyle, heptadécyle, octadécyle, oléyle, nonadécyle, éicosyle, hénéicosyle, docosyle, tricosyle, tétracosyle, pentacosyle, triacontyle, des isomères des précédents, cyclopentyle, cyclohexyle, cycloheptyle, cyclooctyle et cyclododécyle.

4. Procédé selon la revendication 3, dans lequel R₂ est sec-butyle.

5. Procédé selon la revendication 1, dans lequel le nitrophénol est choisi dans le groupe constitué de 2,6-dinitro-4-méthylphénol, 2-nitro-4-méthylphénol, 2,4-dinitro-1-naphtol, 2,4,6-trinitrophénol (acide picrique), 2,4-dinitro-6-méthylphénol, 2,4-dinitrophénol, 2,4-dinitro-6-sec-butylphénol, 4-cyano-2-nitrophénol, 3-iodo-4-cyano-5-nitrophénol, *m-*nitro-*p*-crésol et 2,6-dinitro-*p-*crésol.

6. Procédé selon la revendication 1 pour inhiber la polymérisation prématurée et la croissance de polymères de monomères à insaturation éthylénique, comprenant l'addition auxdits monomères d'une quantité efficace d'une combinaison de
(A) au moins un inhibiteur qui est un phénol sulfoné de formule : 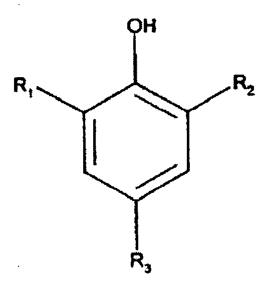 dans laquelle
(1) R₂ est choisi dans le groupe constitué de l'hydrogène et d'un groupe hydrocarbyle ; et
(2) R₁ et R₃ sont indépendamment choisis dans le groupe constitué de l'hydrogène et de SO₃H, sous réserve qu'au moins l'un de R₁ et de R₃ soit SO₃H ;
(B) au moins un inhibiteur qui est un nitrophénol ;
(C) au moins un inhibiteur choisi dans le groupe constitué de composés nitroxyle et de nitrosoanilines ; et
(D) au moins une amine.

7. Procédé selon la revendication 6, dans lequel R₂ est un groupe alkyle à chaîne linéaire ou à chaîne ramifiée ou un groupe alcényle ayant de 1 à 50 atomes de carbone.

8. Procédé selon la revendication 6, dans lequel R₂ est choisi dans le groupe constitué des groupes méthyle, éthyle, propyle, butyle, pentyle, hexyle, heptyle, octyle, 2-éthylhexyle, nonyle, décyle, undécyle, dodécyle, tridécyle, tétradécyle, pentadécyle, hexadécyle, heptadécyle, octadécyle, oléyle, nonadécyle, éicosyle, hénéicosyle, docosyle, tricosyle, tétracosyle, pentacosyle, triacontyle, des isomères des précédents, cyclopentyle, cyclohexyle, cycloheptyle, cyclooctyle et cyclododécyle.

9. Procédé selon la revendication 8, dans lequel R₂ est sec-butyle.

10. Procédé selon la revendication 6, dans lequel le nitrophénol est choisi dans le groupe constitué de 2,6-dinitro-4-méthylphénol, 2-nitro-4-méthylphénol, 2,4-dinitro-1-naphtol, 2,4,6-trinitrophénol (acide picrique), 2,4-dinitro-6-méthylphénol, 2,4-dinitrophénol, 2,4-dinitro-6-sec-butylphénol, 4-cyano-2-nitrophénol, 3-iodo-4-cyano-5-nitrophénol, *m-*nitro-*p-*crésol et 2,6-dinitro-*p-*crésol.

11. Procédé selon la revendication 6, dans lequel (C) est un composé nitroxyle stable stériquement encombré ayant la formule de structure : 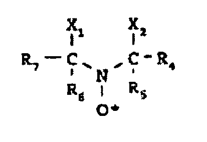 dans laquelle R₄ et R₇ sont indépendamment choisis dans le groupe constitué de l'hydrogène, d'un groupe alkyle et d'un groupe alkyle substitué par un hétéroatome et R₅ et R₆ sont indépendamment choisis dans le groupe constitué d'un groupe alkyle et d'un groupe alkyle substitué par un hétéroatome ; et X₁ et X₂ (1) sont indépendamment choisis dans le groupe constitué d'un atome d'halogène, d'un groupe cyano, COOR₇, -S-COR₇, -OCOR₇, (dans lequel R₇ représente un groupe alkyle ou aryle), amido, -S-C₆H₅, carbonyle, alcényle, ou alkyle ayant de 1 à 15 atomes de carbone, ou (2) pris ensemble, forment une structure de noyau avec l'azote.

12. Procédé selon la revendication 6, dans lequel (C) est une nitrosoaniline de structure : 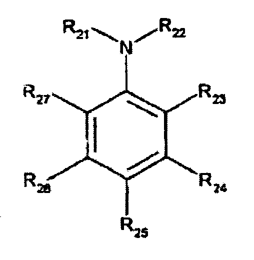 dans laquelle
R₂₁ et R₂₂ sont indépendamment choisis dans le groupe constitué de l'hydrogène, des groupes alkyle, aryle, acyle, hydroxyle, alkoxy, nitroso et sulfonyle, ou R₂₁ et R₂₂ peuvent former un noyau cyclique qui est un groupe aryle, cycloalkyle, polyaryle, ou hétérocyclique ;
R₂₃ à R₂₇ sont indépendamment choisis dans le groupe constitué de l'hydrogène, des groupes alkyle, aryle, acyle, hydroxyle, alkoxy, acyloxy, NR₂₈ (R₂₉), nitro, nitroso, halogène et sulfonyle, ou l'un quelconque de deux R adjacents peut former un noyau cyclique qui est un groupe aryle, cycloalkyle, polyaryle, ou hétérocyclique, sous réserve qu' au moins l'un de R₂₃ à R₂₇ soit un groupe nitroso ; et
R₂₈ et R₂₉ sont indépendamment choisis dans le groupe constitué de l'hydrogène, des groupes alkyle, aryle, acyle et nitroso.

13. Procédé selon la revendication 6, dans lequel (D) est choisi dans le groupe constitué d'α-naphtylamine, thiodiarylamines, *p-*phénylènediamine, o-phénylènediamine, 2,4-diaminodiphénylamine, cyclohexyl-naphtylamine, polybutylamines, méthylaniline, diphényl-*p-*phénylènediamine, phényl-β-naphtylamine, isopropoxy-diphénylamine, aldol-α-naphtylamine, di-β-naphtyl-*p-*phénylènediamine symétrique, triméthyldihydroquinoléine, ditolylamines, phényl-α-naphtylamine, phényl-β-naphtylamine, diaminophénol, 4-cyclohexylaminophénol, *p-*aminophénol, o-aminophénol, et 5-amino-2-hydroxytoluène.

14. Composition comprenant une combinaison de
(A) au moins un inhibiteur qui est un phénol sulfoné de formule : 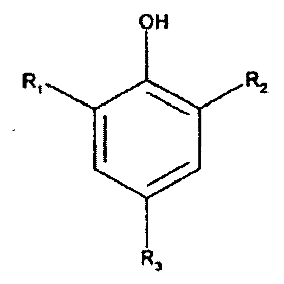 dans laquelle
(1) R₂ est choisi dans le groupe constitué de l'hydrogène et d'un groupe hydrocarbyle ; et
(2) R₁ et R₃ sont indépendamment choisis dans le groupe constitué de l'hydrogène et de SO₃H, sous réserve qu'au moins l'un de R₁ et de R₃ soit SO₃H ;
(B) un inhibiteur qui est un nitrophénol ;
(C) un inhibiteur choisi dans le groupe constitué de composés nitroxyle et de nitrosoanilines ; et
(D) une amine.

15. Composition selon la revendication 14, dans laquelle R₂ est un groupe à chaîne linéaire ou à chaîne ramifiée ou un groupe alcényle ayant de 1 à 50 atomes de carbone.

16. Composition selon la revendication 14, dans laquelle R₂ est choisi dans le groupe constitué des groupes méthyle, éthyle, propyle, butyle, pentyle, hexyle, heptyle, octyle, 2-éthylhexyle, nonyle, décyle, undécyle, dodécyle, tridécyle, tétradécyle, pentadécyle, hexadécyle, heptadécyle, octadécyle, oléyle, nonadécyle, éicosyle, hénéicosyle, docosyle, tricosyle, tétracosyle, pentacosyle, triacontyle, des isomères des précédents, cyclopentyle, cyclohexyle, cycloheptyle, cyclooctyle et cyclododécyle.

17. Composition selon la revendication 14, dans laquelle le nitrophénol est choisi dans le groupe constitué de 2,6-dinitro-4-méthylphénol, 2-nitro-4-méthylphénol, 2,4-dinitro-1-naphtol, 2,4,6-trinitrophénol (acide picrique), 2,4-dinitro-6-méthylphénol, 2,4-dinitrophénol, 2,4-dinitro-6-sec-butylphénol, 4-cyano-2-nitrophénol, 3-iodo-4-cyano-5-nitrophénol, *m-*nitro-*p-*crésol et 2,6-dinitro-*p-*crésol.

18. Composition selon la revendication 14, dans laquelle (C) est un composé nitroxyle stable stériquement encombré ayant la formule de structure : 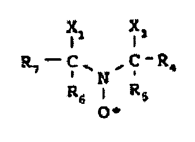 dans laquelle R₄ et R₇ sont indépendamment choisis dans le groupe constitué de l'hydrogène, d'un groupe alkyle et d'un groupe alkyle substitué par un hétéroatome et R₅ et R₆ sont indépendamment choisis dans le groupe constitué d'un groupe alkyle et d'un groupe alkyle substitué par un hétéroatome ; et X₁ et X₂ (1) sont indépendamment choisis dans le groupe constitué d'un atome d'halogène, d'un groupe cyano, COOR₇, -S-COR₇, -OCOR₇, (dans lequel R₇ représente un groupe alkyle ou aryle), amido, -S-C₆H₅, carbonyle, alcényle, ou alkyle ayant de 1 à 15 atomes de carbone, ou (2) pris ensemble, forment une structure de noyau avec l'azote.

19. Composition selon la revendication 14, dans laquelle (C) est une nitrosoaniline de structure : 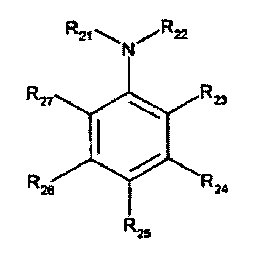 dans laquelle
R₂₁ et R₂₂ sont indépendamment choisis dans le groupe constitué de l'hydrogène, des groupes alkyle, aryle, acyle, hydroxyle, alkoxy, nitroso et sulfonyle, ou R₂₁ et R₂₂ peuvent former un noyau cyclique qui est un groupe aryle, cycloalkyle, polyaryle, ou hétérocyclique ;
R₂₃ à R₂₇ sont indépendamment choisis dans le groupe constitué de l'hydrogène, des groupes alkyle, aryle, acyle, hydroxyle, alkoxy, acyloxy, NR₂₈ (R₂₉) , nitro, nitroso, halogène et sulfonyle, ou l'un quelconque de deux R adjacents peut former un noyau cyclique qui est un groupe aryle, cycloalkyle, polyaryle, ou hétérocyclique, sous réserve qu'au moins l'un de R₂₃ à R₂₇ soit un groupe nitroso ; et
R₂₈ et R₂₉ sont indépendamment choisis dans le groupe constitué de l'hydrogène, des groupes alkyle, aryle, acyle et nitroso.

20. Composition selon la revendication 14, dans laquelle (D) est choisi dans le groupe constitué d'α-naphtylamine, thiodiarylamines, *p-*phénylènediamine, o-phénylènediamine, 2,4-diaminodiphénylamine, cyclohexyl-naphtylamine, polybutylamines, méthylaniline, diphényl-*p-*phénylènediamine, phényl-β-naphtylamine, isopropoxy-diphénylamine, aldol-α-naphtylamine, di-β-naphtyl-*p-*phénylènediamine symétrique, triméthyldihydroquinoléine, ditolylamines, phényl-α-naphtylamine, phényl-β-naphtylamine, diaminophénol, 4-cyclohexylaminophénol, *p-*aminophénol, o-aminophénol, et 5-amino-2-hydroxytoluène.
